# EUROPEAN PATENT SPECIFICATION

(11) **EP 0 349 219 B1**
(45) Date of publication and mention of the grant of the patent: **05.10.1994**
(21) Application number: 89306404.8
(22) Date of filing: 23.06.1989
(51) Int. Cl.: C07D 487/22, G03G 5/04

(54) **1,2,4,5-benzoylenebis(naphtho[1,8-de]pyrimidine) compounds and their use**
1,2,4,5-Benzoylenbis(naphtho[1,8-de]pyrimidin)-Verbindungen und deren Verwendung
Composés 1,2,4,5-benzoylènebis(naphtho[1,8-de]pyrimidiniques) et leur utilisation

(30) Priority: 25.06.1988 JP 157246/88
(43) Date of publication of application: 03.01.1990
(73) Proprietor: SHARP KABUSHIKI KAISHA, Osaka 545 (JP)
(72) Inventor: Kojima, Yoshimi, Nara-shi Nara-ken (JP); Katayama, Satoshi, Nara-shi (JP); Shimoda, Yoshihide, Nara-shi Nara-ken (JP); Sugimura, Hiroshi, Habikino-shi Osaka (JP); Imada, Eiji, Nara-shi Nara-ken (JP)
(74) Representative: West, Alan Harry

(56) References cited:
- EP-A- 0 320 200
- EP-A- 0 320 201
- DE-A- 2 148 101
- PATENT ABSTRACTS OF JAPAN, Unexamined Applications, P field, vol. 9, no. 48, February 28, 1985, The Patent Office Japanese Government, page 47 P 338

## Description

The present invention relates to pyrimidine compounds and their use. More particularly, it relates to 1,2,4,5-benzoylenebis (naphtho[1,8-de] pyrimidine) compounds, which are organic photoconductive substances, and to photosensitive members containing them.

Generally, electrophotography using a photoconductive photosensitive member is one information recording means utilizing photoconductive phenomena.

In electrophotography, the surface of the photosensitive member is uniformly charged by corona discharge or the like, in darkness and then, by subjecting the charged surface to image exposure, the electric charge of the exposed portion is discharged selectively to form an electrostatic latent image on the unexposed portions of the surface. The latent image is converted into a visual image by allowing charged colored fine particles (toner) to adhere to the latent image by electrostatic attraction. The basic properties required for photoconductive photosensitive members used in electrophotographic techniques of these serial processes are:
(1) that the photosensitive members can be uniformly electrified in darkness at a suitable electric potential,
(2) that they have a high electric charge-retentivity in darkness and discharge of the electric charge from them is minor,
(3) that they are superior in photosensitivity and quickly discharge the electric charge by photoirradiation, and the like. Moreover, it is required for the photoconductive photosensitive members to have good stability and endurance; such as, that the surface of them can be discharged easily, with a minor residual electric potential on the surface; that they have mechanical strength and are superior in flexibility; that their electric properties, especially electrification property and remaining electric potential, do not fluctuate even when they are used repeatedly; and that they are resistant to heat, light, temperature, humidity and ozone deterioration.

Photosensitive members for electrophotography now used practically may be divided broadly into two groups, one utilizing inorganic materials and one utilizing organic materials.

As representative photosensitive inorganic members, there can he mentioned those of selenium such as amorphous selenium (a-Se) and amorphous arsenic selenide (a-As₂Se₃), those comprising color-sensitized zinc oxide (ZnO) or cadmium sulfide (CdS) dispersed in a bonding resin, and those using amorphous silicon (a-Si). As representative photosensitive organic members, there are those using a charge-transfer complex of 2,4,7-trinitro-9-fluorenone (TNF) and poly-N-vinylcarbazole (PVK).

These photosensitive members have many merits, but at the same time some drawbacks. For instance, photosensitive members of selenium and photosensitive members using CdS have doubtful heat-resistance and can-stability. Moreover, there is a restriction in the use of those photosensitive members that, because of their toxicity, they cannot be simply thrown away but have to be recovered. Photosensitive members comprising ZnO dispersed in resin are now practically not used, because of their low sensitivity and inferior endurance. Amorphous silicon photosensitive members having such advantages as high sensitivity and high endurance, have also the drawbacks that their manufacturing cost is high because of their complicated manufacturing process, that they give faulty images resulting from faults in the membrane which is inherent in amorphous silicon. Further, they have the disadvantages that their flexibility is not satisfactory, and that their processing into various forms such as drums and sheets is not easy.

In contrast, organic photosensitive members have gained in importance because suitable organic materials without the problems on can-stability and toxicity can be selected from existing various materials, and organic materials having improved durability and low cost have become available. However, the sensitivity of organic photosensitive materials remains to be improved. The PVK-TNF charge-transfer complex series resulted from such an improvement, but did not attain sufficient sensitivity. In addition, various sensitizing methods have been proposed. At present, the main type of organic photosensitive members practically used is laminate type photosensitive members (hereinafter called "multi layer type photosensitive members") having a superior sensitizing property, which comprise a layer (hereinafter called "charge-generating layer") containing a substance which allows generation of charge-carriers by photoirradiation (hereinafter called "charge-generating substance") and a layer (hereinafter called "charge-transporting layer") consisting mainly of a substance which accepts and transports the charge carriers generated in the charge-generating layer (hereinafter called "charge-transporting substance").

As organic materials which may be used for the charge-generating layer, there are known bis-azo pigments such as chlorodianblue, polycyclic quinone series pigments such as dibromoanthanthrone, perillene, quinacridone or phthalocyanine series compounds and azulenium salts. However, some of these charge-generating materials are lacking in photoconductivity though they have a good absorption spectral property for photosensitive members, in the visual ray region, and some of them have an absorption spectral property unsuitable for photosensitive members though they have a good photoconductivity. Thus, it was very difficult to compose a photosensitive member having a good sensitivity, by striving for coexistence of the absorption spectral property and the photoconductivity. The multilayer type photosensitive members provided with such a charge-generating layer which have been hitherto proposed are, for example,
① those wherein a thin layer formed by application of an organic amine solution of chlorodianblue is used as the charge-generating layer and a hydrazone compound is used as the charge-transporting substance of the charge-transporting layer (Japanese Patent Publication No. SHO 55-42380 is referred to),
② those wherein a bis-azo compound is used as the charge-generating substance of the charge-generating layer and a hydrazone compound is used as the charge-transporting layer (Japanese Patent Application Laid-open No. SHO 59-214035 is referred to),
③ those wherein an azulenium salt compound is used as the charge-generating substance of the charge-generating layer and a hydrazone compound or the like is used as the charge-transporting layer (Japanese Patent Application Laid-open No. SHO 59-53850 is referred to),
④ those wherein a perillene derivative is used as the charge-generating substance of the charge-generating layer and an oxadiazole derivative is used as the charge-transporting layer (U.S.P. No. 3871882 is referred to), and the like.

However, these photosensitive members ① - ④ still had insufficient sensitivity, as they were used in practice. Moreover, these hitherto known multilayer type photosensitive members had a problem in their stability when they were used repeatedly.

Therefore, notwithstanding the above-mentioned many merits of the organic photoconductive substances, compared with inorganic ones, they were not widely used for photosensitive members for electrophotography because photosensitive members using them were inferior in sensitivity and endurance.

The present invention seeks to provide novel organic photoconductive compounds and high sensitivity photosensitive members for electrophotography which contain those compounds.

According to the invention, there are now provided compounds of the general formulae (I) and (II) 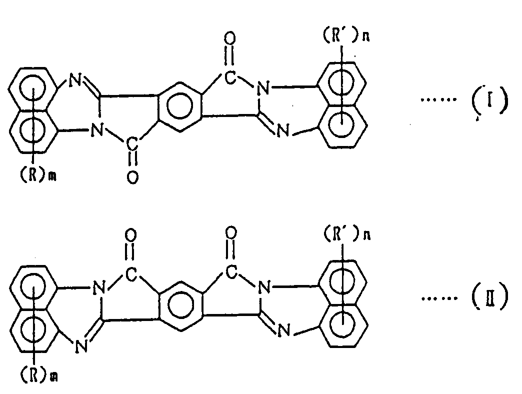
in which each of R and R' is a halogen atom or a hydroxy, nitro or cyano group an alkyl or alkoxy group which may be substituted by a halogen atom or a hydroxy, nitro or cyano group an aryl or aralkyl group which may be substituted by a halogen atom or a hydroxy, nitro or cyano group or a C₁-C₅ alkyl, C₁-C₅ alkoxy or di(C₁-C₅) alkylamino group a carboxy group which may be salified or esterified by a C₁-C₅ alkyl group or an aryl group which may be substituted in the manner specified above or a carbamoyl group which may be substituted by an alkyl or alkoxy group which may be substituted in the manner specified above, an aryl group which may have one or more halogen, nitro or cyano substituents, or a carbazolyl or dibenzofuranyl group, and m and n each is an integer from 1 to 6.

The present invention provides also photosensitive members which comprise a photosensitive layer containing such a compound on a conductive support. Such photosensitive members according to the invention have an extremely high sensitivity and are superior in photoconductivity compared with convention photosensitive members, and can be used not only in electrophotography, such as laser printers and CRT printers as well as copying machines, but also widely in solar batteries and photosensors.

The corresponding unsubstituted bis-pyrimidine compounds i.e. those compounds of the formulae (I) and (II) in which both R and R' are hydrogen, and their preparation, are described in DE-A-2148101. However, those compounds are described in that document in the context only of their use as pigment dyestuffs.

The invention is described below in greater detail by way of example only with reference to the accompanying drawings, in which:
Figures 1 - 8 are enlarged sectional views of photosensitive members of the present invention; and
Figures 9 and 10 are an IR absorption spectrum and a visible absorption spectrum of a representative pyrimidine compound (1,2,4,5-Benzoylenebis(naphtho[1,8-de]pyrimidine), respectively.

In the general formulae (I) and (II), each of R and R' is a halogen atom or a hydroxy, nitro or cyano group an alkyl or alkoxy group which may be substituted by a halogen atom or a hydroxy, nitro or cyano group an aryl or aralkyl group which may be substituted by a halogen atom or a hydroxy, nitro or cyano group or a C₁-C₅ alkyl, C₁-C₅ alkoxy or di(C₁-C₅) alkylamino group a carboxy group which may be salified or esterified by a C₁-C₅ alkyl group or an aryl group which may be substituted in the manner specified above or a carbamoyl group which may be substituted by an alkyl or alkoxy group which may be substituted in the manner specified above, an aryl group which may have one or more halogen, nitro or cyano substituents or a carbazolyl or dibenzofuranyl group, and m and n each is an integer from 1 to 6.

The halogen atoms may be fluorine, chlorine, bromine or iodine atoms.

The alkyl groups include straight and branched chain alkyl groups having from one to five carbon atoms, such as methyl, ethyl, n-propyl and isopropyl groups. The alkyl groups may carry one or more substituents, such as halogen atoms and hydroxy, nitro, and cyano groups. Chlorine atoms and nitro groups are the preferred substituents.

The alkoxy groups include straight and branched chain alkoxy groups having from one to five carbon atoms, such as methoxy, ethoxy, n-propoxy and isopropoxy groups. The alkoxy group may carry one or more substituents, as described above for the alkyl group. Chlorine atoms and nitro groups are the preferred substituents.

The aryl groups include phenyl and naphthyl groups. The aryl group may carry one or more substituents, such as halogen atoms and nitro cyano hydroxy, C₁-C₅ alkyl, C₁-C₅ alkoxy and di-C₁-C₅ alkylamino groups. The C₁-C₅ alkyl groups may have straight or branched chains.

The aralkyl groups include benzyl and phenethyl groups. The aryl moiety may carry one or more substituents, as described above for the aryl group.

The carboxyl group may be in the form of an ester or salt. The former may be a C₁-C₅ alkyl or aryl ester. The latter may be a sodium or potassium salt. Examples of ester groups are methoxy carbonyl-, phenoxy carbonyl-, and phenoxy carbonyl- substituted as described above for the aryl groups.

Examples of the substituents for the carbamoyl groups are alkyl, alkoxy, aryl, and heteroaromatic carbazolyl and dibenzofuranyl groups. The alkyl and alkoxy groups may be substituted as described above. The aryl group includes phenyl, naphthyl and anthryl groups. It may also carry one or more substituents, as described above.

In the pyrimidine compounds of the present invention, each of m and n is preferably 1 or 2 when each of R and R' is a halogen atom, and 1 when each of R and R' is other than a halogen atom.

The pyrimidine compounds of the present invention may be prepared by utilizing known processes. For example, they may be prepared by treating pyromellitic anhydride of the following formula (III) with an aromatic diamine of the following general formulae (IV) and (V) (in which R, R', m and n have the meanings specified above) in an inert solvent, at an elevated temperature, preferably at 100 - 350°C, particularly a temperature higher than 200°C. The reaction is carried out to completion. A reaction time of 3 - 24 hours is usually preferred (Bull. Chem. Soc. Japan, 25, 411 - 413 (1952); ibid. 27, 602 - 605 (1954) and referred to).

Examples of inert solvents are nitrobenzene, aprotic polar solvents such as N-methylpyrrolidone, N,N-dimethylformamide and N,N-dimethylacetamide, basic solvents such as quinoline, and chlorinated solvents such as o-dichlorobenzene, and mixtures of such solvents. 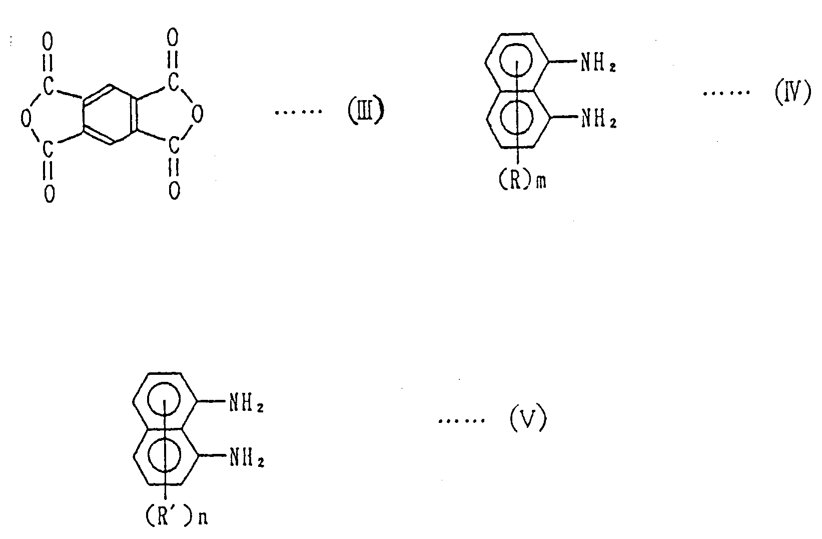
According to the above method, pyrimidine compounds represented by the general formula (I) (trans-isomer) and formula (II) (cis-isomer) are obtained in the form of mixtures. The pyrimidine compounds of the present invention include the trans-and cis-isomers and mixtures thereof. It is possible to separate the isomers by conventional methods if necessary. The mixture, however, is sufficient for use as a photoconductive material.

Examples of pyrimidine compounds of the present invention are set out below, only the trans-isomers being shown. The known, unsubstituted compound is included as compound (1) for the purposes of reference and completeness. 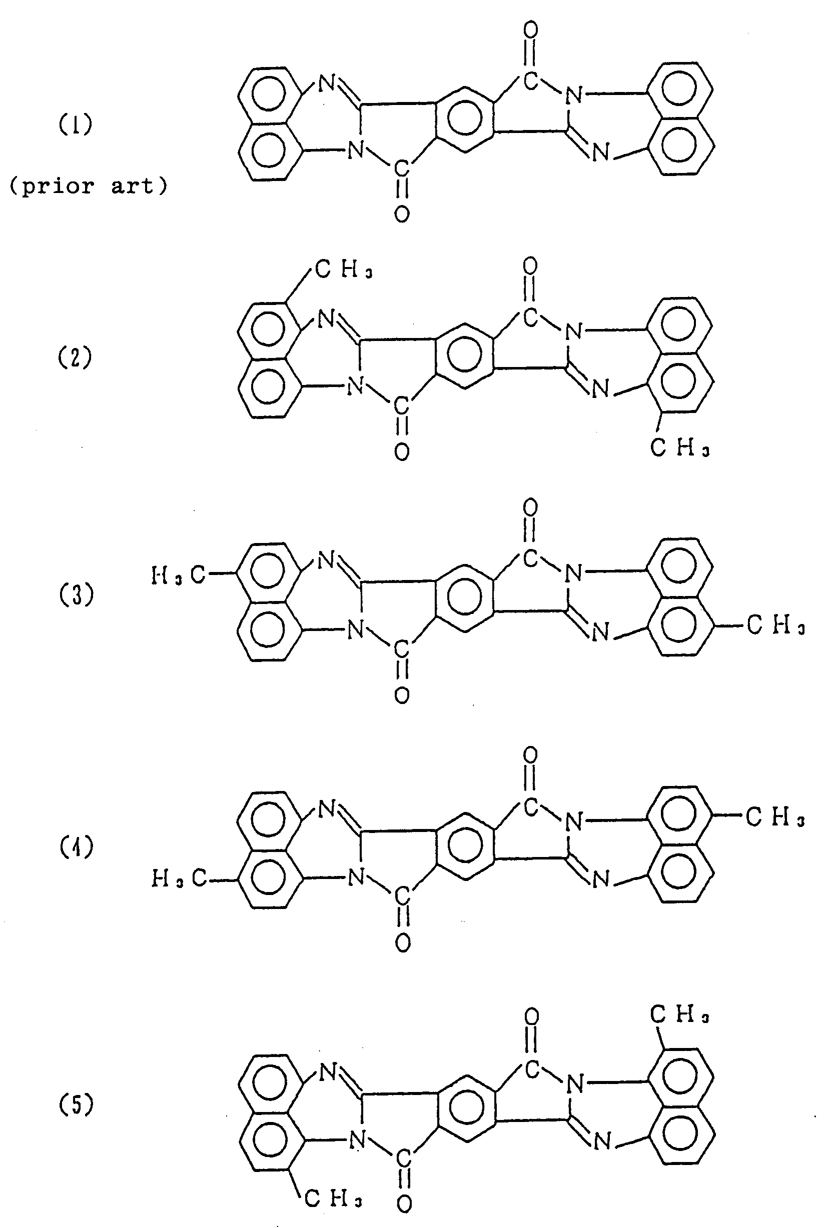 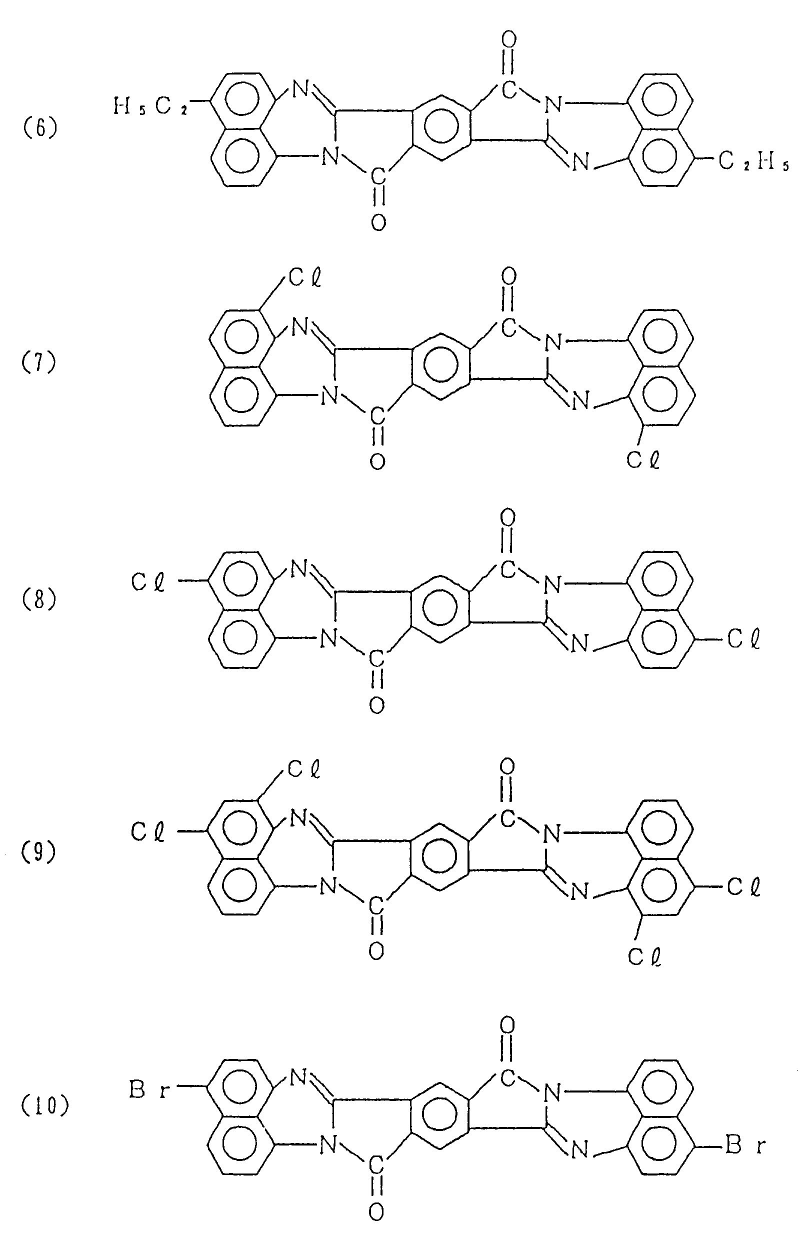 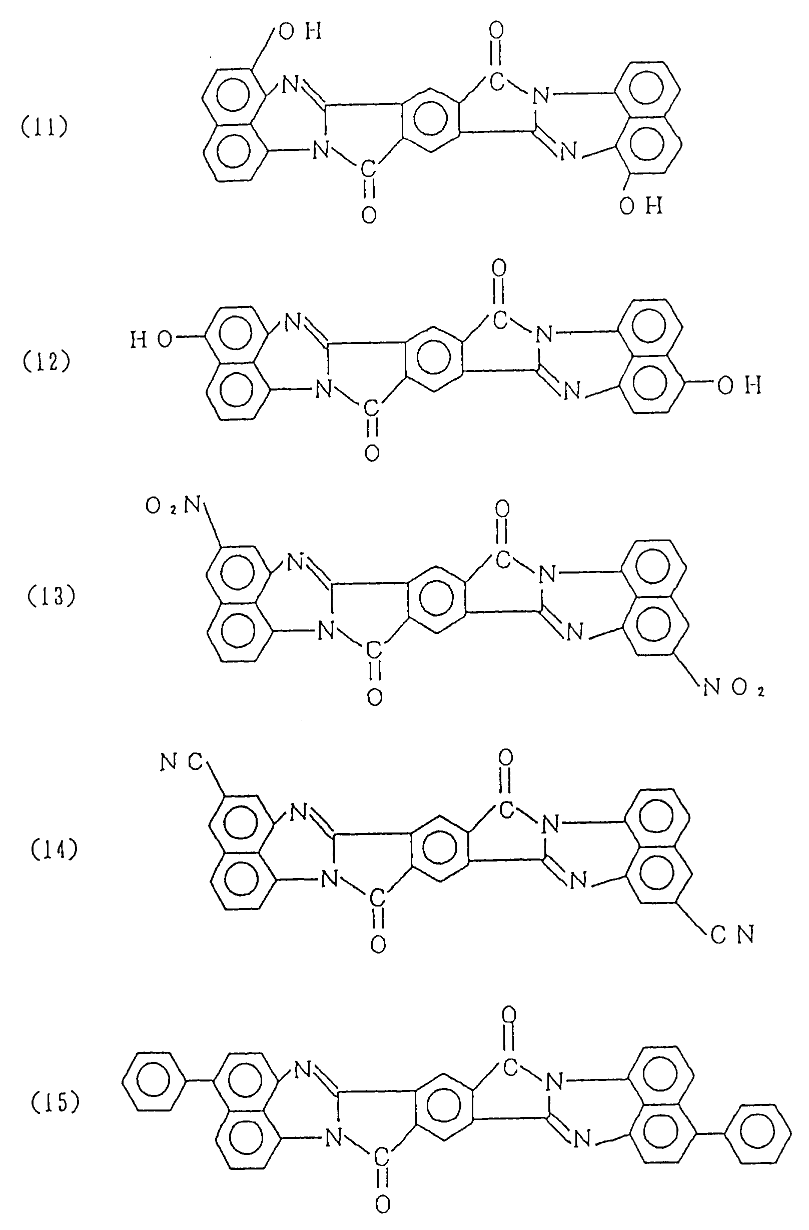 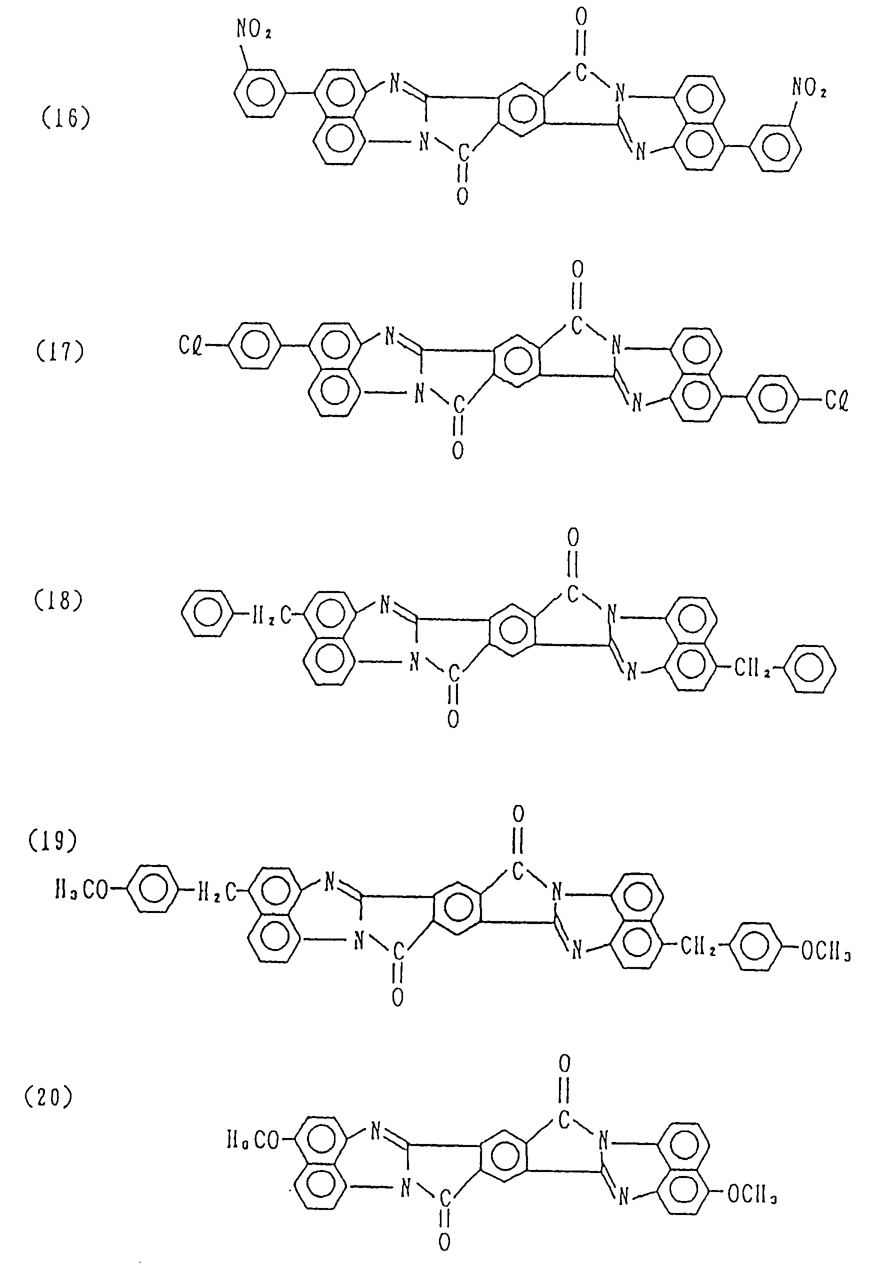 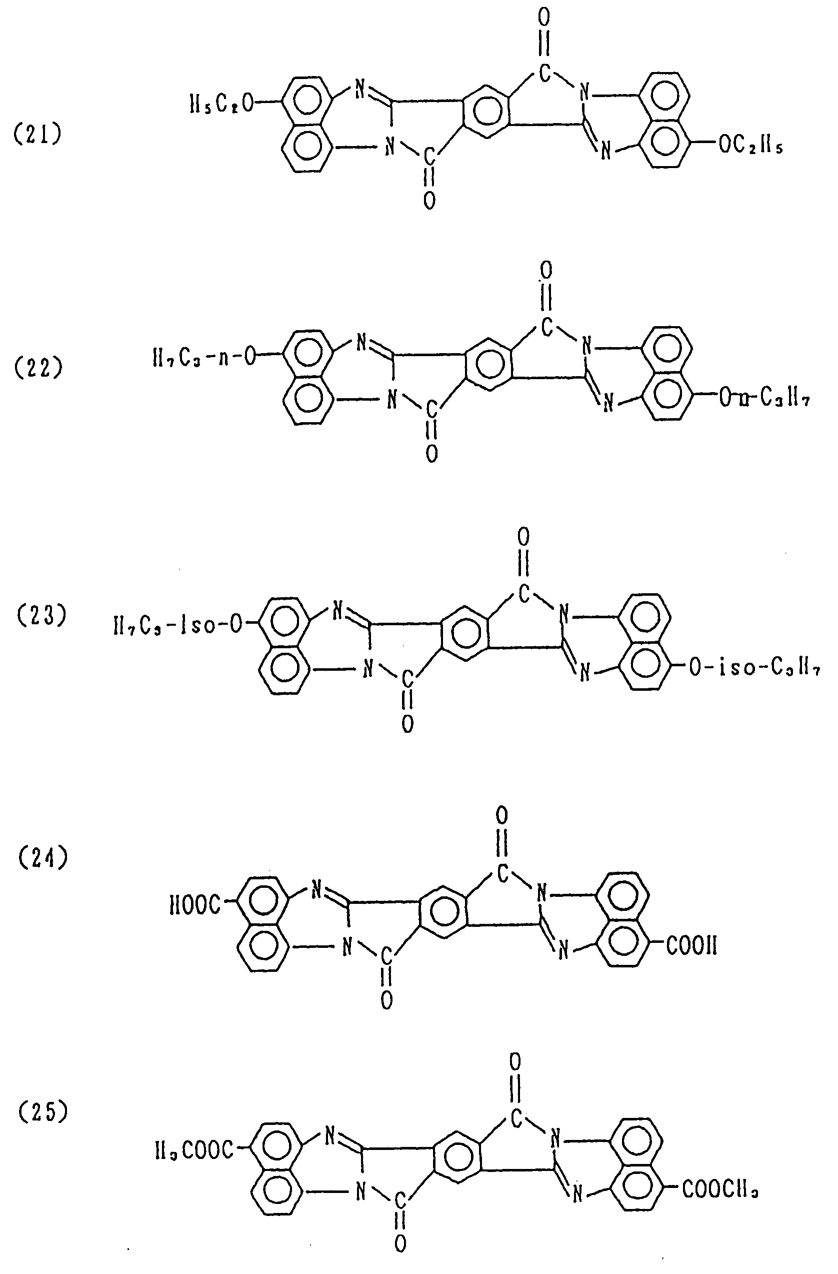 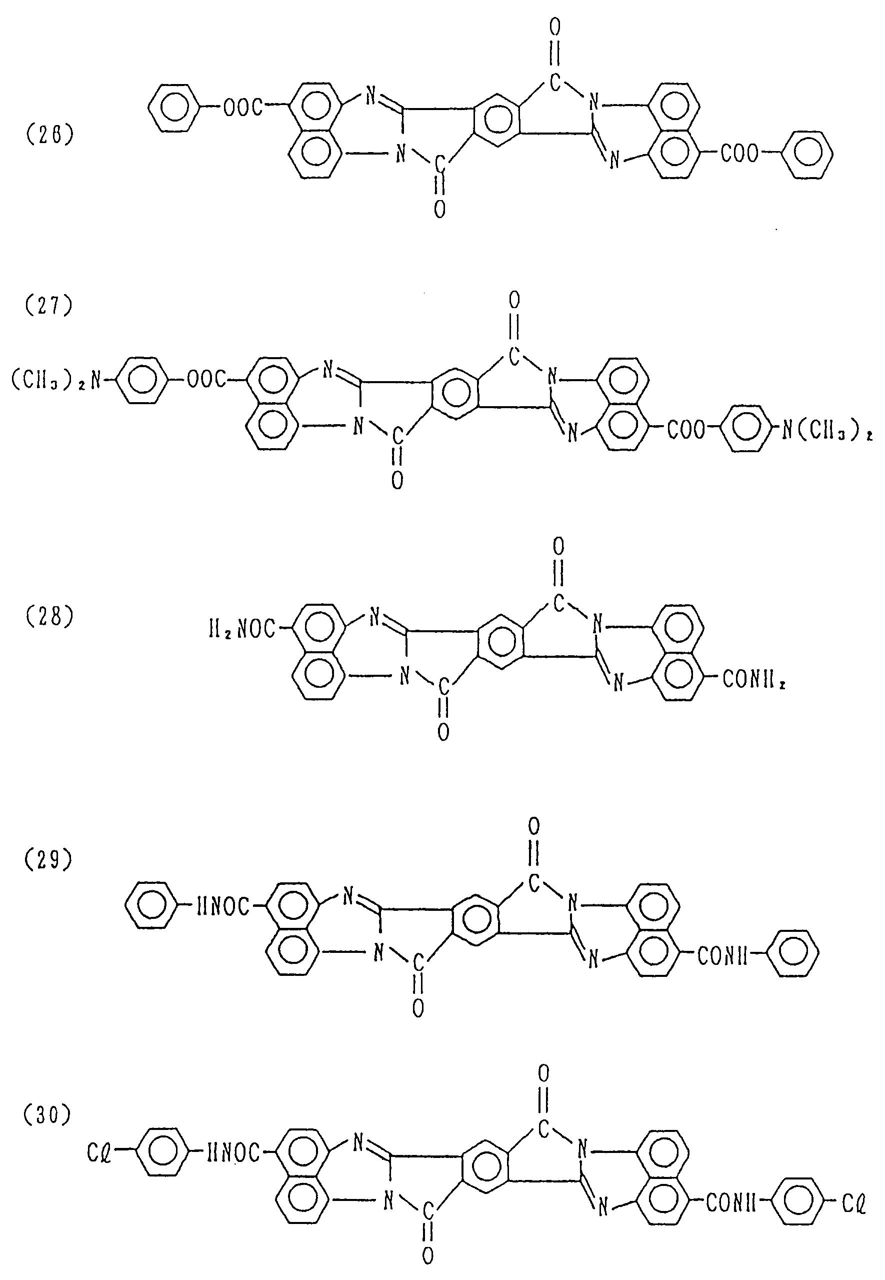 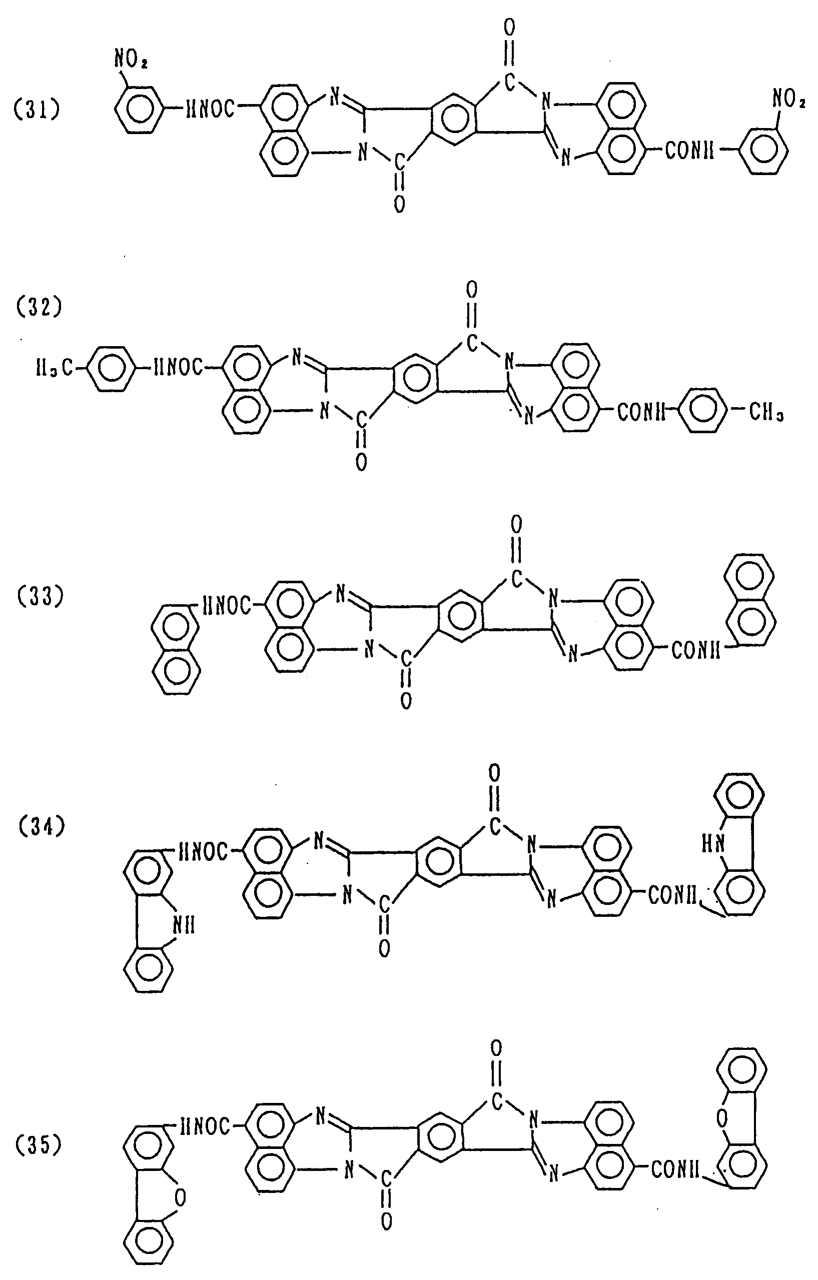
The pyrimidine compounds represented by the above formulae (I) and (II) are novel compounds having photoconductivity. Thus, the present invention provides also photosensitive members, which especially have excellent properties for electrophotography. The members can be prepared by disposing on a conductive support one or more photosensitive layers containing either a compound of the formula (I) or a compound of the formula (II), or the mixture of the compounds of the formulae (I) and (II). Various types of the photosensitive layers can be considered.

For example, the pyrimidine compounds of the present invention may be dispersed in a polymer binder and applied on a conductive support, to make a photosensitive layer. In another process, the pyrimidine compounds of the present invention may be utilized as a charge-generating substance, since their charge-generating ability is especially superior among their photoconductive properties. That is, the pyrimidine compounds may be dispersed in a binder polymer containing a charge-transporting substance (the binder polymer is not necessarily used when the charge-transporting substances has a coating membrane-forming ability), to form a photosensitive layer. Further, it may be used to prepare the so-called multilayer type photosensitive members for electrophotography having a multilayer type photosensitive layer which comprises a laminate of a charge-generating layer containing the pyrimidine compounds and a charge-transporting layer.

Suitable conductive supports are those substrates which per se have conductivity, such as aluminum, aluminum alloy, copper, zinc, stainless steel, nickel, chromium and titanium, plastics having a conductive coating membrane layer formed by a vacuum evaporation method or the like, of aluminum, aluminum alloy, indium oxide or tin oxide, substrates comprising plastics, paper, or the like, impregnated with conductive particles or plastics having conductive polymers.

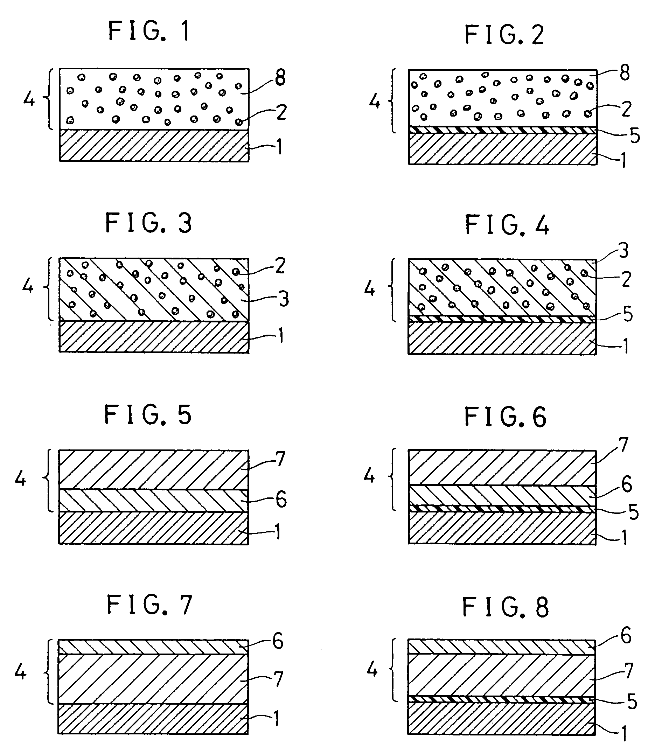

Figures 1 - 8 show representative forms of the photosensitive members for electrophotography of the present invention. In the photosensitive members of Fig. 1 and Fig. 2, the pyrimidine compounds of the present invention act as a photoconductor, and charge-generation and charge-transportation are effected through the pyrimidine compounds. In the Fig. 2, 1 denotes a conductive support, 2 denotes the pyrimidine compound, 4 denotes a photosensitive layer, 5 denotes an undercoating layer and 8 denotes a binder. In Fig. 3 and Fig. 4 are shown those photosensitive members in which a photosensitive layer 4 comprising a dispersion of the pyrimidine compound 2 as the charge-generating substance in a charge-transporting substance-containing layer 3 is provided directly or through an undercoating layer 5 of a conductive support 1. As shown in Fig. 5 - Fig. 8, a multilayer type photosensitive layer 4 comprising a laminate of a charge-generating layer 6 consisting mainly of the pyrimidine compound of the present invention, as the charge-generating substance, and a charge-transporting layer 7 consisting mainly of a charge-transporting substance, may be provided on a conductive support 1 to prepare photosensitive members. An undercoating layer 5 may be interposed between the conductive support 1 and the photosensitive layer 4, also in these cases. Further, as shown in Fig. 5 and Fig. 6 or Fig. 7 and Fig. 8, when the photosensitive layer 4 is composed of two layers, either of the charge-generating layer 6 and the charge-transporting layer 7 may be the upper layer. With a multilayer type photosensitive member having a photosensitive layer 4 composed of two layers, especially superior photosensitive members for electrophotography are obtained.

The pyrimidine compounds of the present invention constituting the photosensitive layer 4 of the laminate type may form the charge-generating layer 6 on the conductive support 1 or on the charge-transporting layer 7, directly or though an intermediate layer such as the undercoating layer 5. The forming of such a charge-generating layer is effected by vacuum evaporation of a pyrimidine compound of the present invention as the charge-generating substance, or by application of a dispersion obtained by dispersing a pyrimidine compound of the present invention is a suitable solvent and, if necessary, mixing with a binder, or of a dispersion obtained by dispersing a pyrimidine compound of the present invention in a suitable solvent containing a binder dissolved therein. Generally speaking, the latter method is preferred.

When the charge-generating layer is prepared by such an application method, dispersion of the pyrimidine compound in a binder solution can be performed efficiently by using a ball mill, sand mill, roll mill, atritor, vibration mill or supersonic disperser, and application of the dispersion can be performed efficiently by using an air doctor coater, blade coater, spray coater, hot coater or squeeze coater.

As the binder used here, there can be mentioned, for example, those insulating resins such as polycarbonate, acrylic, polyester, polystyrene, melamine, silicone, polyvinyl butyral, polyamide, polyarylate phenoxy and copolymer resins containing two or more of the recurring units of the above resins, such as vinyl chloride/vinyl acetate copolymer resin and acrylonitrile/styrene copolymer resins. Any generally used resins can be used alone or as a mixture of two or more of them.

Further, as the solvent for dissolving the binder there can be used, for example, alcohols such as methanol, ethanol and isopropanol; ketones such as acetone, methyl ethyl ketone and cyclohexanone; esters such as ethyl acetate and butyl acetate; ethers such as tetrahydrofuran and dioxane; aromatic hydrocarbons such as benzene, toluene and xylene; and aproptic polar solvents such as N,N-dimethylformamide, N,N-dimethylacetamide and dimethyl sulfoxide.

The membrane thickness of the charge-generating layer 6 thus formed is suitably 0.01 to 20 µm, preferably 0.05 - 5 µm, more preferably 0.1 - 2 µm. Further, it is necessary to granulate the pyrimidine compound 2 of the present invention into fine particles having a particle size of 5 µm or less, preferably 3 µm or less, most preferably 1 µm or less. The binder resin contained in the charge-generating layer is suitably 80% by weight or less, preferably 40% by weight or less.

The charge-transporting layer 7 is electrically connected with the charge-generating layer 6 and has a function of transporting the charge carriers poured in from the charge generating layer, in the presence of an electric field. As materials for such charge-transporting layers, though the materials are generally classified into electron-transporting substances and hole-transporting substances, both and also mixtures of the two can be used for the photosensitive layer of the present invention. As the electron-transporting substances, those substances having electron attractive groups such as nitro groups and cyano groups, for example, nitrated fluorenones such as 2,4,7-trinitro-9-fluorenone and 2,4,5,7-tetranitro-9-fluorenone, and trinitrotoluene are mentioned. As the hole-transporting substances, electron donative substances, for example, heterocyclic compounds such as carbazole, oxazole, thiazole, oxadiazole, imidazole, and pyrazoline, low molecular weight charge-transporting substances such as aniline derivatives, arylamine derivatives, hydrazone derivatives and stilbene derivatives, high molecular weight charge-transporting substantives such as poly-N-vinylcarbazole, halogenated poly-N-vinylcarbazole, polyvinyl pyrene and polyvinyl anthracene, may be used. The charge-transporting substances can be used individually or as a mixture of two or more of them.

When a low molecular weight charge-transporting substance is used, the coating membrane can be formed by selecting a suitable binder. However, a high molecular weight charge-transporting substance having membrane-forming properties may also be used. Further, it is also possible to mix the above-mentioned high molecular weight charge-transporting substance with the binder.

As the binder used here, the above-mentioned various resins used for the preparation of charge-generating layers can be used, and various solvents as described above are appliable as the solvent for the binder.

The membrane thickness of the charge-generating layer thus formed is 2 - 100 µm, preferably 5 - 30 µm.

As the undercoating layer 5, high molecular weight organic substances such as gelatin, casein, polyvinyl alcohol, and ethyl cellulose, and aluminum oxide may be used, besides the high molecular weight polymers used as the above-mentioned binder resin.

The photosensitive layer of the photosensitive members for electrophotography of the present invention may further contain one or more electron accepting substances and coloring materials, for the purpose of improving sensibility, suppressing the elevation of residual electric potential and fatigue, which appear on repeated use.

As the electron accepting substances used here, there can be mentioned, for example, acid anhydrides such as succinic anhydride, maleic anhydride, phthalic anhydride, and 4-chloronaphthalic anhydride; cyano compounds such as tetracyanoethylene and terephthalmalononitrile; aldehydes such as 4-nitrobenzaldehyde; anthraquinones such as anthraquinone and 1-nitroanthraquinone; and polycyclic and heterocyclic nitro compounds such as 2,4,7-trinitrofluorenone and 2,4,5,7-tetranitrofluorenone. They are used as chemical sensitizers.

As the coloring materials, those organic photoconductive compounds such as xanthene dyes, thiazine dyes, triphenylmethane dyes, quinoline pigments and copper phthalocyanine pigments are mentioned. They may be used as optical sensitizers.

Further, the photosensitive layer of the photosensitive members for electrophotography of the present invention may contain known plasticizers, for the purpose of improving molding characteristics, flexibility and mechanical strength. As plasticizer, there can be mentioned dibasic acid esters, fatty acid esters, phosphoric acid esters, phthalic acid esters, chlorinated paraffins and epoxy-type plasticizers. The photosensitive layer may contain, if necessary, anti-oxidants, ultraviolet ray absorbing agents, and the like.

The following Examples illustrate the invention. Example 1 describing the preparation and characterisation of the known unsubstituted pyrimidine compound is included for reference purposes only.

The words "Metalumy"_{,} "Ethulek" and "lupiron" used in the Examples are registered Trade Marks.

### Example 1

In 200 ml of nitrobenzene, 0,474 g (3.0 m mol) of 1,8-diaminonaphthalene was treated with 0.327 g (1.5 m mol) of pyromellitic anhydride at 200°C for 24 hours while stirring. As the reaction proceeded, formation of precipitates was observed. The precipitates were collected by filtration and washed 3 times with 500 ml each of tetrahydrofuran, 3 times with 500 ml each of nitrobenzene, 3 times with 500ml each of diethyl ether, and further 5 times with 2ℓ of acetone. The crystals thus obtained were dried at 60°C under vacuum, whereby 0.56 g of a colored compound was obtained (yield: 70%):

The properties of the compound are as follows.
Melting point : above 300°C

| Elementary analysis (C₃₀H₁₄ O₂N₄) | | |
|---|---|---|
| | Calculated | Found |
| C(%) | 77.9 | 77.6 |
| H(%) | 3.0 | 3.2 |
| N(%) | 12.1 | 11.7 |

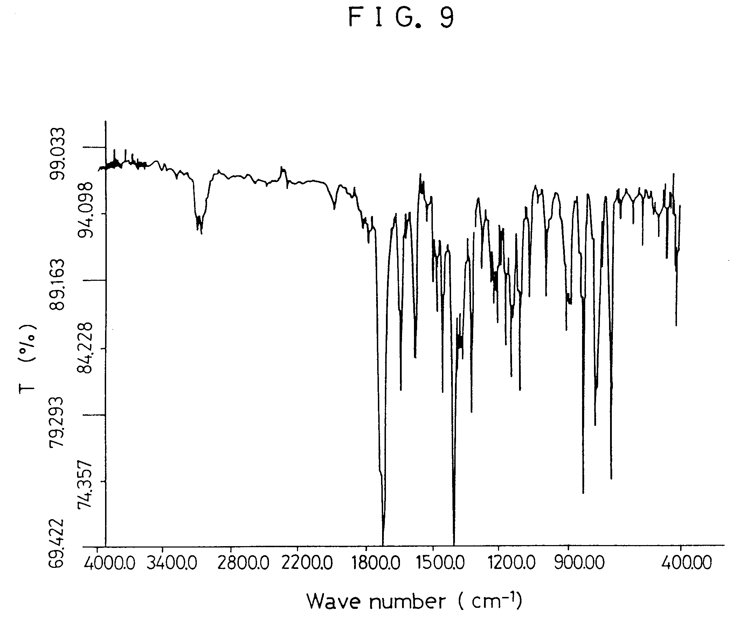

IR absorption spectrum is shown in the IR chart of Fig. 9 obtained with an infrared absorption spectroscopy.

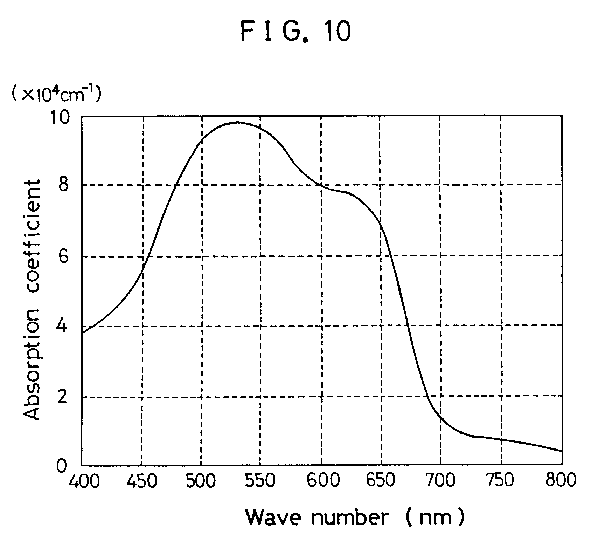

Visual region absorption spectrum is shown in the chart of Fig. 10 obtained by a visible light absorption spectroscopy using as a sample thin phenoxy resin film containing the compound dispersed.

From the above results, the compound was determined to be a mixture of pyrimidine compounds of the following formulae (1) and (2).

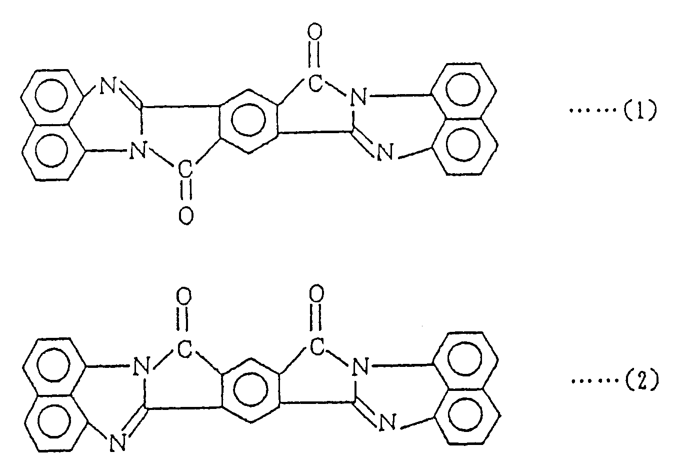

### Example 2

A dispersion obtained by dispersing 2 parts by weight of the pyrimidine compound No. 2, which was prepared from pyromellitic anhydride and 1,8-diamino-7-methyl naphthalene in the same manner as Example 1 and 1 part by weight of a phenoxy resin (pKHH, from Union Carbide Corp.) in 8 parts by weight of dioxane for 12 hours by means of a ball mill disperser, was applied onto the surface of a PET coated with alminum by vacuum evaporation (polyethylene terephthalate : Metalumy #100 from Toray Industries, Inc.) by means of a Baker applicator and dried at 80°C for 1 hour, whereby a photosensitive member of the form shown in Fig. 1 having a photosensitive layer 10 µm thick was prepared.

The photosensitive member thus prepared was corona electrified by means of an electrostatic paper analyzer, Model SP-428 from Kawaguchi Electric Co., Ltd. at +5KV in static mode and, after maintaining in dark place for 5 seconds, exposed at an illumination intensity of 5 lux, to investigate its electrification characteristics. As the electrification characteristics, initial electric potential (V₀), exposure amount necessary for attenuating the potential as attenuated in darkness for 5 seconds the half (E 1/2), residual potential at 5 seconds after the start of exposure (V_{R}) and charge retention coefficient as attenuated in darkness for 5 seconds (V_{K}) were measured. The results obtained are shown in Table 1, together with the results of the following Example 3 - 5.

### Examples 3 - 5

The above described pyrimidine compounds No. 12,15,18 each were synthesized in the same manner as Example 1 except for using 1,8-diamino-5-hydroxynaphthalene(Example 3), 1,8-diamino-5-phenylnaphthalene(Example 4), 1,8-diamino-5-benzylnaphthalene(Example 5), respectively, instead of 1,8-diaminonaphthalene.

Photosensitive members having the form shown in Fig. 2 were prepared in the same manner as Example 2, except that each of the pyrimidine compounds obtained above was used instead of the pyrimidine compound No. 2 and, as the substrate, was used one prepared by forming an undercoating layer of vinyl chloride/vinyl acetate/maleic anhydride copolymer (Ethulek MF-10, from Sekisui Chemical Co., Ltd.) having a thickness of 0.05 µm on the PET coated with aluminum by vacuum evaporation which was used in Example 2. With these photosensitive members, the same measurements as Example 2 were effected. The result obtained are shown in Table 1.

**Table 1**

| Results of measurements by means of the electrostatic paper analyzer | | | | | |
|---|---|---|---|---|---|
| Example | Pyrimidine compound No. | V₀ (V) | E1/2 (lux.sec) | V_{R} (V) | V_{K} (%) |
| 2 | 2 | +376 | 12.0 | + 8 | 94 |
| 3 | 12 | +333 | 14.0 | +11 | 91 |
| 4 | 15 | +385 | 11.0 | +12 | 95 |
| 5 | 18 | +401 | 13.0 | +15 | 98 |

### Example 6

A dispersion obtained by dispersing 2 parts by weight of the pyrimidine compound No. 3, which was prepared in the same manner as Example 1, except for using 1,8-diamino-5-methylnaphthalene instead of 1,8-diamino-naphthalene, 15 parts by weight of 2,4,7-trinitro-9-fluorenone and 15 parts by weight of a polycarbonate resin (Iupiron, from Mitsubishi Gas Chemical Company Inc.) in 188 parts by weight of dichloromethane for 12 hours by means of a ball mill disperser, was applied onto the surface of the PET coated with aluminum by vacuum evaporation (the same one as used in Example 2) by means of a Baker applicator and dried at 80°C for 1 hour, whereby a photosensitive member of the form shown in Fig. 3 having a photosensitive layer 20 µm thick was prepared. Then, the same measurements as Example 2 were effected, except that the corona electrification was performed at -5KV, instead of +5KV in Example 2, in the electrostatic copying test of the phtotsensitive member. The results obtained are shown in Table 2, together with the results of the following Examples 7 - 9.

### Examples 7 - 9

The above described pyrimidine compounds No. 11,13,16 each were synthesized in the same manner as Example 1 except for using 1,8-diamino-7-hydroxynaphthalene (Example 7), 1,8-diamino-6-nitronaphthalene (Example 8), 1,8-diamino-5-(m-nitrophenyl)-naphthalene (Example 9), respectively, instead of 1,8-diamino-naphthalene.

Photosensitive members having the form shown in Fig. 4 were prepared in the same manner as Example 6, except that each of the pyrimidine compounds obtained above was used instead of the pyrimidine compound No. 3 and , as the substrate, was used one prepared by forming an undercoating layer of a vnyl chloride/vinyl acetate/maleic anhydride copolymer having a thickness of 0.05 µm on the PET coated with aluminum by vacuum evaporation which was used in Example 6. With these photosensitive members, the same measurements as Example 6 were effected. The results obtained are shown in Table 2.

**Table 2**

| Results of measurements by means of the electrostatic paper analyzer | | | | | |
|---|---|---|---|---|---|
| Example | Pyrimidine compound No. | V₀ (V) | E 1/2 (lux.sec) | V_{R} (V) | V_{K} (%) |
| 6 | 3 | -412 | 15.0 | -15 | 88 |
| 7 | 11 | -399 | 12.0 | -20 | 89 |
| 8 | 13 | -387 | 11.0 | -17 | 81 |
| 9 | 16 | -376 | 9.0 | -14 | 80 |

### Example 10

A dispersion obtained by dispersing 2 parts by weight of the pyrimidine compound No. 1 prepared in Example 1 and 1 part by weight of a phenoxy resin (the same one as used in Example 2) in 97 parts by weight of 1,4-dioxane for 12 hours by means of a ball mill disperser, was applied onto the same PET coated with aluminum by vacuum evaporation as used in Example 2 by means of a Baker applicator and dried at room temperature for 1 hour, whereby a charge-generating layer 0.5 µm thick was formed.

Next, 1 part by weight of a hydrazone series compound represented by the following formula, 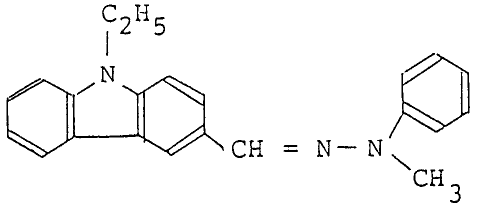
1 part by weight of a polycarbonate resin (the same one as used in Example 6) and 8 parts by weight of dichloromethane were mixed together and dissolved by stirring with a stirrer. The solution thus obtained was applied onto the charge-generating layer by means of a Baker applicator and then dried at 80°C for 1 hour, whereby photosensitive member of the form shown in Fig. 5 having a photosensitive layer 20 µm thick was prepared. The same measurements as Example 6 were effected with regard to this photosensitive member. The results obtained are shown in Table 3, together with the results of the following Examples 11 - 13.

### Examples 11 - 13

The above described pyrimidine compounds No. 10,17,18 each were synthesized in the same manner as Example 1 except for using 1,8-diamino-5-bromonaphthalene (Example 11), 1,8-diamino-5-(p-chlorophenyl)naphthalene (Example 12), 1,8-diamino-5-benzylnaphthalene (Example 13), respectively, instead of 1,8-diaminonaphthalene.

Photosensitive members having the form shown in Fig. 6 were prepared in the same manner as Example 10, except that each of the pyrimidine compounds obtained above was used instead of the pyrimidine compound No. 1 and, as the substrate, was used one prepared by forming an undercoating layer of a vinyl chloride/vinyl acetate/maleic anhydride copolymer (the same one as used in Example 10) having a thickness of 0.05 µm on the PET coated with aluminum by vacuum evaporation which was used in Example 10. With regard to these photosensitive members, the same measurements as Example 10 were effected. The results obtained are shown in Table 3.

**Table 3**

| Results of measurements by means of the electrostatic paper analyzer | | | | | |
|---|---|---|---|---|---|
| Example | Pyrimidine compound No. | V₀ (V) | E 1/2 (lux.sec) | V_{R} (V) | V_{K} (%) |
| 10 | 1 | -721 | 4.0 | - 7 | 96 |
| 11 | 10 | -700 | 5.0 | - 9 | 95 |
| 12 | 17 | -699 | 7.0 | -12 | 90 |
| 13 | 18 | -721 | 9.0 | -17 | 97 |

### Example 14

One part by weight of a hydrazone series compound represented by the following formula 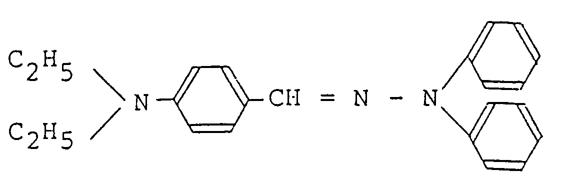
1 part by weight of a polycarbonate resin (the same one as used in Example 6) and 8 parts by weight of dichloromethane were mixed together and dissolved by stirring with a stirrer. The solution thus obtained was applied onto the PET coated with aluminum by vacuum evaporation (the same one as used in Example 2), by means of a Baker applicator, and dried at 80°C for 1 hour, whereby a charge-transporting layer having a membrane thickness of 19.5 µm was formed.

Next, a dispersion obtained by dispersing 2 parts by weight of the pyrimidine compound No. 5 (synthesized in the same manner as Example 1) and 1 part by weight of a phenoxy resin (the same one as used in Example 2) in 97 parts by weight of 1,4-dioxane for 12 hours by means of a ball mill disperser, was applied onto the charge-transporting layer by means of a Baker applicator, and then a photosensitive member of the form shown in Fig. 7 having a photosensitive layer 20 µm thick was prepared. With regard to this photosensitive member, the same measurements as Example 2 were effected. The results obtained are shown in Table 4, together with the results of the following Examples 15 - 17.

### Examples 15 - 17

The above described pyrimidine compounds No. 6,8,14 each were synthesized in the same manner as Example 1 except for using 1,8-diamino-5-ethylnaphthalene (Example 15), 1,8-diamino-5-chloronaphthalene (Example 16), 1,8-diamino-5-cyanonaphthalene (Example 17), respectively, instead of 1,8-diaminonaphthalene.

Photosensitive members having the form shown in Fig. 8 were prepared in the same manner as Example 14, except that each of the pyrimidine compounds obtained above was used instead of the pyrimidine compound No. 5 and, as the substrate, was used one prepared by forming an undercoating layer of a vinyl chloride/vinyl acetate/maleic anhydride copolymer (the same one as used in Example 14) having a thickness of 0.05 µm on the PET coated with aluminum by vacuum evaporation which was used in Example 14. With regard to these photosensitive members, the same measurements as Example 14 were effected. The results obtained are shown in Table 4.

**Table 4**

| Results of measurements by means of the electrostatic paper analyzer | | | | | |
|---|---|---|---|---|---|
| Example | Pyrimidine compound No. | V₀ (V) | E 1/2 (lux.sec) | V_{R} (V) | V_{K} (%) |
| 14 | 5 | +698 | 9.0 | +11 | 89 |
| 15 | 6 | +678 | 9.0 | + 9 | 81 |
| 16 | 8 | +700 | 7.0 | +17 | 86 |
| 17 | 14 | +704 | 8.0 | +16 | 89 |

### Comparative Example 1

A photosensitive member was prepared according to the same manner as Example 2, except that a polycyclic quinone series pigment (Monolite Red 2Y, from I.C.I. Ltd.) of the formula 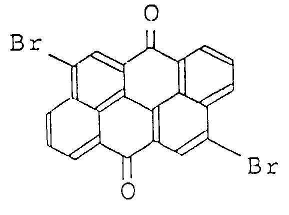
was used instead of the pyrimidine compound No. 2. Then, the same measurements as example 2 were effected with regard to the photosensitive member. The results obtained are shown in Table 5.

### Comparative Example 2

A photosensitive member was prepared according to the same manner as Example 3, except that a quinacridone series pigment (Cinquasia Red Y RT-759-D, from Ciba-Geigy Ltd.) of the formula 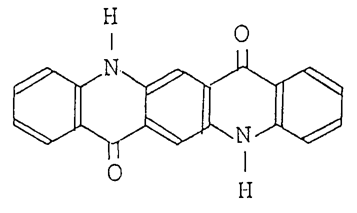
was used instead of the pyrimidine compound No. 12. The same measurements as Example 3 were effected with regard to the photosensitive member. The results obtained are shown in Table 5.

### Comparative Example 3

A photosensitive member was prepared according to the same manner as Example 6, except that an isoindolinone series pigment (Irgazin Yellow 2RLT, from Ciba-Geigy Ltd.) of the formula 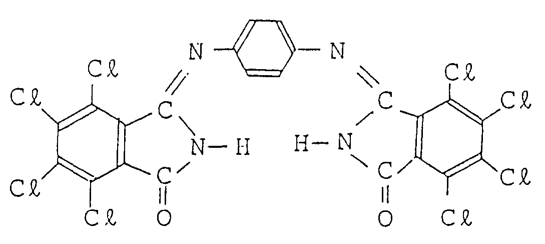
was used instead of the pyrimidine compound No. 3. The same measurements as Example 6 were effected with regard to the photosensitive member. The results obtained are shown in Table 5.

### Comparative Example 4

A photosensitive member was prepared according to the same manner as Example 7, except that an indigo series pigment (Vat Blue 1, from Mitsui Toatsu Dyestuff Inc.) of the formula 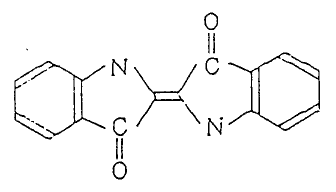
was used instead of the pyrimidine compound No. 11. The same measurements as example 7 were effected wiht regard to the photosensitive member. The results obtained are shown in Table 5.

### Comparative Example 5

A photosensitive member was prepared according to the same manner as Example 10, except that a thioindigo series pigment (Vat Red 41, from Mitsui Toatsu Dyestuff Inc.) of the formula 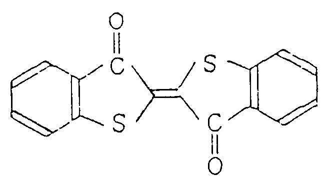
was used instead of the pyrimidine compound No. 1. The same measurements as Example 10 were effected with regard to the photosensitive member. The results obtained are shown in Table 5.

### Comparative Example 6

A photosensitive member was prepared accoding to the same manner as Example 12, except that a xanthene series dyestuff (Eosine Y) of the formula 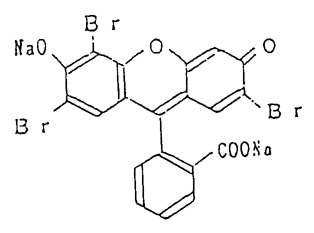
was used instead of the pyrimidine compound No. 17. The same measurements as Example 12 were effected with regard to the photosensitive member. The results obtained are shown in Table 5.

### Comparative Example 7

A photosensitive member was prepared according to the same manner as Example 14, except that an acridine series dyestuff (Acridine Yellow, from Chroma Ltd.) of the formula 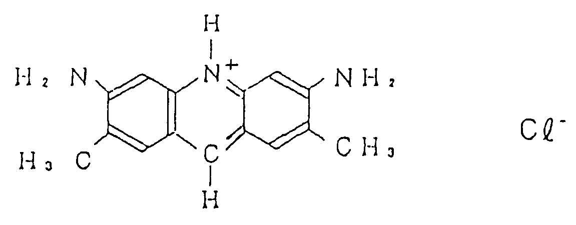
was used instead of the pyrimidine compound No. 5. The same measurements as Example 14 were effected with regard to the photosensitive member. The results obtained are shown in Table 5.

### Comparative Example 8

A photosensitive member was prepared according to the same manner as Example 17, except that a triphenylmethane series dyestuff (Ethylviolet) of the formula 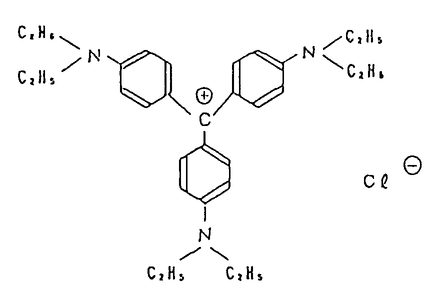
was used instead of the pyrimidine compound No. 14. The same measurements as Example 17 were effected with regard to the photosensitive member. The results obtained are shown in Table 5.

**Table 5**

| Results of measurements by means of the electrostatic paper analyzer | | | | |
|---|---|---|---|---|
| Comparative Example | V₀ (V) | E 1/2 (lux.sec) | V_{R} (V) | V_{K} (%) |
| 1 | + 200 | 54 | + 45 | 80 |
| 2 | + 300 | 39 | + 55 | 75 |
| 3 | - 365 | 89 | - 50 | 70 |
| 4 | - 390 | 32 | - 60 | 75 |
| 5 | - 430 | 40 | - 30 | 80 |
| 6 | - 405 | 33 | - 35 | 81 |
| 7 | + 355 | 45 | + 40 | 76 |
| 8 | + 370 | 42 | + 45 | 77 |

## Claims

1. A compound of the general formula (I) or (II): 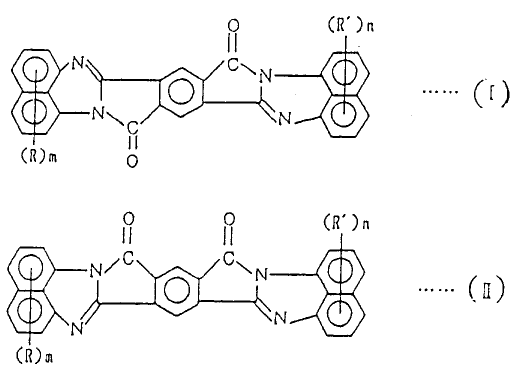 in which each of R and R' is
a halogen atom or a hydroxy, nitro or cyano group
an alkyl or alkoxy group which may be substituted by a halogen atom or a hydroxy, nitro or cyano group
an aryl or aralkyl group which may be substituted by a halogen atom or a hydroxy, nitro or cyano group or a C₁-C₅ alkyl, C₁-C₅ alkoxy or di(C₁-C₅) alkylamino group
a carboxy group which may be salified or esterified by a C₁-C₅ alkyl group or an aryl group which may be substituted in the manner specified above or
a carbamoyl group which may be substituted by an alkyl or alkoxy group which may be substituted in the manner specified above, an aryl group which may have one or more halogen, nitro or cyano substituents, or a carbazolyl or dibenzofuranyl group,
and m and n each is an integer from 1 to 6.

2. A compound according to claim 1, wherein each of R and R' is a chlorine or bromine atom.

3. A compound according to claim 1, wherein each of R and R' is a methyl or ethyl group which may be substituted in the manner specified in claim 1.

4. A compound according to claim 1, wherein each of R and R' is a methoxy, ethoxy or propoxy group which may be substituted in the manner specified in claim 1.

5. A compound according to claim 1, wherein each or R and R' is a phenyl group which may be substituted in the manner specified in claim 1.

6. A compound according to claim 1, wherein each of R and R' is a benzyl group which may be substituted by a C₁-C₅ alkoxyl group.

7. A photosensitive member comprising a photosensitive layer containing a compound according to any one of claims 1 to 6, on a conductive support.

8. A photosensitive member according to claim 7, wherein the photosensitive layer is of the multilayer type and consists of a charge-generating layer and a charge-transporting layer, the former containing the compound according to any one of claims 1 to 6.

9. A photosensitive member according to claim 8, wherein the charge-generating layer has a thickness of 0.01 - 20 µm.

10. A photosensitive member according to claim 8 or claim 9, wherein the charge-transporting layer has a thickness of 2 - 100 µm.

## Patentansprüche

1. Verbindung der allgemeinen Formel (I) oder (II) 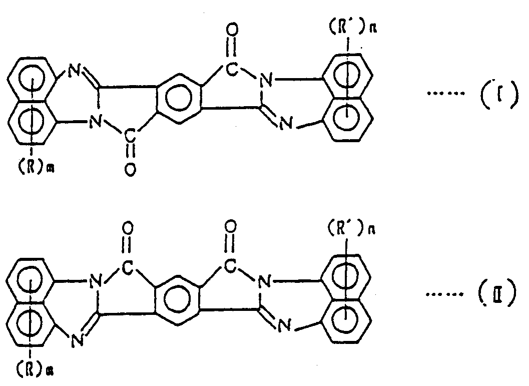 worin R und R' jeweils bedeuten:
ein Halogenatom oder eine Hydroxy-, Nitro- oder Cyanogruppe,
eine Alkyl- oder Alkoxygruppe, welche durch ein Halogenatom oder eine Hydroxy-, Nitro- oder Cyanogruppe substituiert sein kann,
eine Aryl- oder Aralkylgruppe, welche durch ein Halogenatom oder eine Hydroxy-, Nitro- oder Cyanogruppe oder eine C₁-C₅-Alkyl-, C₁-C₅-Alkoxy- oder Di(C₁-C₅)-Alkylaminogruppe substituiert sein kann,
eine Carboxygruppe, welche ein Salz gebildet haben kann oder verestert sein kann durch eine C₁-C₅-Alkylgruppe oder eine Arylgruppe, welche in der oben genannten Weise substituiert sein kann, oder
eine Carbamoylgruppe, welche substituiert sein kann durch eine Alkyl- oder Alkoxygruppe, welche in der oben angegebenen Weise substituiert sein kann, eine Arylgruppe, welche einen oder mehrere Halogen-, Nitro- oder Cyanosubstituenten aufweisen kann, oder eine Carbazolyl- oder Dibenzofuranylgruppe,
und m und n jeweils eine ganze Zahl von 1 bis 6 bedeuten.

2. Verbindung nach Anspruch 1, wobei jedes R und R' ein Chlor- oder Bromatom ist.

3. Verbindung nach Anspruch 1, wobei jedes R und R' eine Methyl- oder Ethylgruppe ist, welche in der in Anspruch 1 angegebenen Weise substituiert sein kann.

4. Verbindung nach Anspruch 1, wobei jedes R und R' eine Methoxy-, Ethoxy- oder Propoxygruppe ist, welche in der in Anspruch 1 angegebenen Weise substituiert sein kann.

5. Verbindung nach Anspruch 1, wobei jedes R und R' eine Phenylgruppe ist, welche in der in Anspruch 1 angegebenen Weise substituiert sein kann.

6. Verbindung nach Anspruch 1, wobei jedes R und R' eine Benzylgruppe ist, welche durch eine C₁-C₅-Alkoxylgruppe substituiert sein kann.

7. Lichtempfindliches Element, umfassend auf einem leitfähigen Träger eine lichtempfindliche Schicht, welche ein Verbindung nach mindestens einem der Ansprüche 1 bis 6 enthält.

8. Lichtempfindliches Element nach Anspruch 7, wobei die lichtempfindliche Schicht vom Mehrfachschicht-Typ ist und aus einer ladungserzeugenden Schicht und einer ladungstransportierenden Schicht besteht, wobei die erstere die Verbindung nach mindestens einem der Ansprüche 1 bis 6 enthält.

9. Lichtempfindliches Element nach Anspruch 8, wobei die ladungserzeugende Schicht eine Dicke von 0,01-20 µm besitzt.

10. Lichtempfindliches Element nach Anspruch 8 oder Anspruch 9, wobei die ladungstransportierende Schicht eine Dicke von 2- 100 µm besitzt.

## Revendications

1. Composé de formule générale (I) ou (II) : 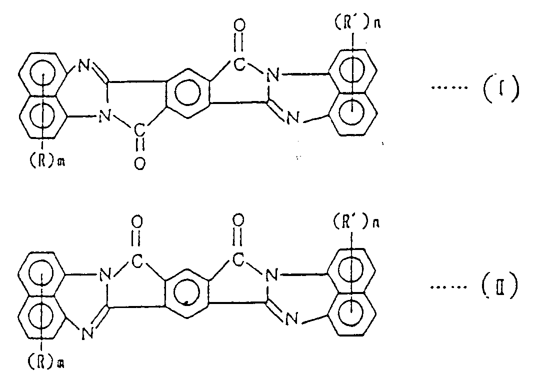 dans laquelle chacun des groupes R et R' est :
un atome d'halogène ou un groupe hydroxy, nitro ou cyano,
un groupe alkyle ou alcoxy qui peut être substitué par un atome d'halogène ou un groupe hydroxy, nitro ou cyano,
un groupe aryle ou aralkyle qui peut être substitué par un atome d'halogène ou un groupe hydroxy, nitro ou cyano, ou un groupe alkyle en C₁-C₅, alcoxy en C₁-C₅ ou di(alkyl en C₁-C₅) amino,
un groupe carboxy qui peut être salifié ou estérifié par un groupe alkyle en C₁-C₅ ou un groupe aryle qui peut être substitué de la manière spécifiée ci-dessus,
un groupe carbamoyle qui peut être substitué par un groupe alkyle ou alcoxy qui peut être substitué de la manière spécifiée ci-dessus,
un groupe aryle qui peut avoir un ou plusieurs substituents halogène, nitro ou cyano, ou un groupe carbazolyle ou dibenzofuranyle.
et m et n sont chacun un entier de 1 à 6.

2. Composé selon la revendication 1, dans lequel chacun des groupes R et R' est un atome de chlore ou de brome.

3. Composé selon la revendication 1, dans lequel chacun des groupes R et R' est un groupe méthyle ou éthyle qui peut être substitué de la manière spécifiée dans la revendication 1.

4. Composé selon la revendication 1, dans lequel chacun des groupes R et R' est un groupe méthoxy, éthoxy ou propoxy qui peut être substitué de la manière spécifiée dans la revendication 1.

5. Composé selon la revendication 1, dans lequel chacun des groupes R et R' est un groupe phényle qui peut être substitué de la manière spécifiée dans la revendication 1.

6. Composé selon la revendication 1, dans lequel chacun des groupes R et R' est un groupe benzyle qui peut être substitué par un groupe alkoxy en C₁ - C₅.

7. Elément photosensible comprenant une couche photosensible, contenant un composé selon l'une quelconque des revendications 1 à 6, sur un support conducteur.

8. Elément photosensible selon la revendication 7, dans lequel la couche photosensible est du type à couches multiples et est constituée d'une couche génératrice de charges et d'une couche de transfert de charges, la première couche contenant le composé selon l'une quelconque des revendications 1 à 6.

9. Elément photosensible selon la revendication 8, dans lequel la couches génératrice de charges a une épaisseur de 0,01 à 20 µm.

10. Elément photosensible selon la revendication 8 ou 9, dans lequel la couche de tranfert de charges a une épaisseur de 2 à 100 µm.
